# EUROPÄISCHE PATENTANMELDUNG

(11) **EP 1 025 854 A1**
(43) Veröffentlichungstag der Anmeldung: **09.08.2000**
(21) Anmeldenummer: 00102135.1
(22) Anmeldetag: 07.02.2000
(51) Int. Cl.: A61K 39/395, A61P 37/06

(54) **Verwendung depletorisch oder mitogen wirkender Antikörper gegen CD3/TCR in der Immuntherapie**

(30) Priorität: 08.02.1999 DE 19905048
(71) Anmelder: GSF-Forschungszentrum für Umwelt und Gesundheit GmbH, 85764 Neuherberg (DE)
(72) Erfinder: Kummer, Udo, Dr., 80538 München (DE)
(74) Vertreter: Bösl, Raphael, Dr. rer. nat., Dipl.-Chem.

(57) **Zusammenfassung**

Die vorliegende Erfindung bezieht sich auf die Verwendung von einem oder mehreren IgG Antikörpern in der Immuntherapie, wobei der oder die Antikörper immer dann Zielzellen effizient eliminieren, wenn er oder sie gegen Antigene auf vorzugsweise einer Zelloberfläche gerichtet ist oder sind und im wesentlichen mit den Fcγ-Rezeptoren I, II und III zu interagieren vermag/vermögen. Des weiteren bezieht sich die vorliegende Erfindung auf die Verwendung eines oder mehrerer IgG Antikörper mit Spezifität für Signal-übertragende Moleküle auf Zelloberflächen, die im wesentlichen ausschließlich mit dem hoch-affinen Fcγ-Rezeptoren II und III interagieren und im wesentlichen die Zellen aktivieren und ihre Teilung in Gang setzen. Einbezogen in die vorliegende Erfindung ist auch die Verwendung eines oder mehrerer Antikörper gegen mindestens einen Fcγ-Rezeptor in der Immuntherapie, wobei in Gegenwart des oder der genannten Antikörper eine vorwiegend eliminatorische Aktivität eines entsprechenden IgG Antikörpers in eine vorwiegend aktivierende Aktivität umgewandelt wird.

## Beschreibung

Die vorliegende Erfindung bezieht sich auf die Verwendung von einem oder mehreren IgG Antikörpern in der Immuntherapie, wobei der oder die Antikörper immer dann Zielzellen effizient eliminieren, wenn er oder sie gegen Antigene auf vorzugsweise einer Zelloberfläche gerichtet ist oder sind und im wesentlichen mit den Fcγ-Rezeptoren I, II und III zu interagieren vermag/vermögen. Des weiteren bezieht sich die vorliegende Erfindung auf die Verwendung eines oder mehrerer IgG Antikörper mit Spezifität für Signal-übertragende Moleküle auf Zelloberflächen, die im wesentlichen ausschließlich mit dem hoch-affinen Fcγ-Rezeptoren II und III interagieren und im wesentlichen die Zellen aktivieren und ihre Teilung in Gang setzen. Einbezogen in die vorliegende Erfindung ist auch die Verwendung eines oder mehrerer Antikörper gegen mindestens einen Fcγ-Rezeptor in der Immuntherapie, wobei in Gegenwart des oder der genannten Antikörper eine vorwiegend eliminatorische Aktivität eines entsprechenden IgG Antikörpers in eine vorwiegend aktivierende Aktivität umgewandelt wird.

Es ist bekannt, daß Zellen des adaptiven Immunsystems spezifische Moleküle über Rezeptoren oder Rezeptorkomplexe erkennen, was wiederum die Aktivierung der Zelle zur Folge hat. Ein Beispiel eines Rezeptorkomplexes ist der T-Zellrezeptorkomplex (TCR/CD3), der für die Erkennung eines Peptid-Antigens, des mit Hilfe des sogenannten 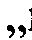 major histocompability complex (MHC)" auf der Oberfläche aller kernhaltigen Körperzellen (MHC Klasse I) oder auf bestimmten Immunzellen (MHC Klasse II) präsentiert wird, verantwortlich ist. Der T-Zellrezeptorkomplex wird ausschließlich auf der Oberfläche von T-Zellen exprimiert und besteht aus der Antigen-bindenden Einheit (αβ- oder γδ-Heterodimer), die mit dem CD3 Komplex assoziiert ist. Der CD3 Komplex besteht seinerseits aus zwei Heterodimeren (CD3γε- und CD3δε-Einheit) und dem ξξ Homodimer (Bentley, GA & Mariuzza, RA (1996) Annu Rev Immunol 14:563-590). Das Homodimer ist überwiegend für die Signaltransduktion verantwortlich. (Alberola-Ila, J.et al. (1997) Annu Rev Immunol 15:725-154).

Die Stimulierung des TCR/CD3-Komplexes führt zur Aktivierung der T-Zelle und nachfolgend zu einer Antigen-spezifischen Immunantwort. (Janeway, CA, Jr. (1992) Annu Rev Immunol 10:645-674; Weiss, A & Littmann, DR (1994) Cell 67:263-274).

Des weiteren ist bekannt, daß die Belegung von Signal-übertragenden Oberflächenmolekülen, sogenannte Triggermoleküle wie z.B. der TCR/CD3-Komplex, mit spezifischen Antikörpern zur Auslösung eines Aktivierungssignals in der Zielzelle führt, wenn FcγR-tragende Effektorzellen für den initialen Aggregationsprozeß des Oberflächenmoleküls anwesend sind. (Kann, EAR et al. (1986) Cell Immunol 98:181-187; Hirsch, R. et al. (1989) J Immunol 142:737-743). Anti-TCR/CD3-Antikörper können so die Interaktion des T-Zellrezeptors mit dem MHC-komplexierten Antigen nachahmen, wobei die T-Zellen aktiviert und ihre Teilung (Proliferation) in Gang gesetzt wird. Dieser Effekt wird als mitogene Aktivität des Antikörpers bezeichnet.

Überraschenderweise konnten mehrere anti-TCR/CD3 IgG Antikörper identifiziert werden, die ohne wesentliche mitogene Aktivität ihre Zielzellen vorwiegend physikalisch eliminieren (depletorische Aktivität des Antikörper). In der voliegenden Erfindung konnte nun nachgewiesen werden, daß die depletorische Aktiviät solcher Antikörper immer dann dominiert, wenn sie mit dem gesamten Spektrum der Fcγ-Rezeptoren, also mit den Fcγ-Rezeptoren FcγRI, FcγRII und FcγRIII zu interagieren vermögen. Dagegen dominiert die mitogene Aktivität solcher Antikörper immer dann, wenn sie von diesem Interaktionsmuster abweichen, also nur mit den niedrig-affinen Fcγ-Rezeptoren II und III oder ausschließlich mit dem hoch-affinen Fcγ-Rezeptor I interagieren. Die letztgenannte Situation kann durch die Gegenwart von Antikörpern herbeigeführt werden, die gegen die Ligandenbindungsstelle der niedrig-affinen Fcγ-Rezeptoren (FcγRII und FcγRIII) gerichtet sind und so deren Funktion blockieren. Eine derartige Manipulation führt dazu, daß ursprünglich depletorisch wirkende anti-TCR/CD3 IgG Antikörper mitogene Aktivität aufweisen.

Im allgemeinen unterscheidet man die Fc-Rezeptoren, d. h. die Fcγ- und Fcε-Rezeptoren in hoch-affine und niedrig-affine Rezeptoren. Der Begriff hoch-affin bedeutet, daß die entsprechenden Rezeptoren (Fcγ-Rezeptor I und Fcε-Rezeptor I) monomeres Immunglobulin stabil, d. h. mit hoher Affinität binden können, während die niedrig-affinen Rezeptoren (Fcγ-Rezeptoren II und III und Fcε-Rezeptor II) nur aggregiertes bzw. komplexiertes, d. h. zellgebundenes Immunglobulin nachweisbar binden können (Daeron, M. (1997) Annu. Rev. Immunol. 15:203-234). Affinitätsbestimmungen der verschiedenen Fc-Rezeptoren für Immunglobuline können gemäß Hulett M.D. & Hogarth, P.M. (1994) Adv. Immunol., 57, 1 - 127 durchgeführt werden.

Während die variablen Abschnitte der leichten und schweren Ketten eines Antikörpers die Stelle ausbilden, die an das Antigen bindet, legt der konstante Fc-Teil die Klassen bzw. Subklassenzugehörigkeit eines Antikörpers fest und verleiht ihnen spezifische biologische Eigenschaften. So werden über den Fc-Bereich eines Antikörpers nach dessen Bindung in einem weiteren Schritt sekundäre oder Effektor-Funktionen, wie z.B. die Interaktion mit Fcγ-Rezeptoren, vermittelt. Die Zugehörigkeit des IgG Antikörpers zu einer bestimmten Subklasse ist im Hinblick auf diese Effektorfünktion eine wichtige Größe (Daeron, M. (1997) Annu. Rev. Immunol. 15:203-234). Dieser Zusammenhang stimmt mit den im Rahmen dieser Erfindung gemachten Beobachtungen überein.

Durch die Wahl geeigneter Antikörper bzw. Antikörpermischungen, die in ihrer Spezifität und IgG Subklasse eindeutig definiert sind, ist es gemäß der vorliegenden Erfindung möglich auf besonders einfache Art und Weise eine selektive Immuntherapie zu etablieren und dabei funktionelle Aktivitäten von Zellpopulationen gezielt zu manipulieren.

So kann beispielsweise durch die Wahl der geeigneten IgG-Antikörperspezifität und -subklasse, zum einen die depletorische Aktivität der Antikörper genutzt werden. Zum anderen kann man auch auf die mitogene Aktivität von entsprechenden Antikörpern zurückgreifen, wenn das gesamte Immunsystem oder Teile davon aktiviert werden sollen, beispielsweise bei der Verstärkung der Hämatopoese bei myelodysplastischen Erkrankungen. Auf die depletorische Aktivität des Antikörpers wird vorzugsweise immer dann zurückgegriffen, wenn unerwünschte Immunreaktionen, wie sie z.B. bei klinischen Gewebe- oder Organtransplantationen auftreten, effektiv supprimiert werden sollen.

Ein Gegenstand der vorliegenden Erfindung ist daher die Verwendung eines oder mehrerer IgG-Antikörper in der Immuntherapie beispielsweise für die Herstellung eines Arzneimittels, wobei der oder die Antikörper gegen Antigene auf einer Zelloberfläche gerichtet sind, im wesentlichen mit den Fcγ-Rezeptoren I, II und III interagieren und eine im wesentlichen depletorische Aktivität zeigen. Die Oberflächenantigene sind üblicherweise für bestimmte Zellpopulationen bzw. Subpopulationen spezifisch, wie z.B. der TCR/CD3-Komplex für die T-Zellen als bevorzugtes Beispiel eines Signal-übertragenden Antigens auf der Zelloberfläche.

Im vorliegenden Beispiel sind das die Antikörper 7D6(DSM ACC2382), 17A2(DSM ACC2380) und/oder H57-597(DSM ACC2384), welche bei der DSMZ-Deutsche Sammlung von Mikroorgansmen und Zellkulturen GmbH, Mascheroder Weg 1b, D-38124 Braunschweig am 17.12.1998 (7D6 und 17A2) und am 13.1.1999 (H57-597) in Form ihrer Hybridome hinterlegt wurden.

Ein weiterer Gegenstand der vorliegenden Erfindung ist die Verwendung eines oder mehrerer IgG-Antikörper in der Immuntherapie beispielsweise für die Herstellung eines Arzneimittels, wobei der oder die Antikörper im wesentlichen mit den Fcγ-Rezeptoren II und III oder im wesentlichen mit dem Fcγ-Rezeptor I interagieren und gegen Signal-übertragende Oberflächenmoleküle, insbesondere gegen den TCR/CD3-Komplex, gerichtet sind. Die genannten Antikörper besitzen eine im wesentlichen mitogene Aktivität und sind vorzugsweise die Antikörper 145-2C11(DSM ACC2378) und/oder KT3(DSM ACC2381), die ebenso jeweils am 17.12.1998 bei der DSMZ hinterlegt wurden.

Ein weiterer Gegenstand der vorliegenden Erfindung ist die Verwendung eines oder mehrerer Antikörper gegen mindestens einen Fcγ-Rezeptor in der Immuntherapie, beispielsweise zur Herstellung eines Arzneimittels, wobei der oder die genannten Antikörper in Anwesenheit von depletorisch wirkenden Antikörpern eine im wesentlichen mitogene Aktivität der depletorisch wirkenden IgG-Antikörper verursachen. Bevorzugte Beispiele von im wesentlichen depletorisch wirkenden Antikörpern sind bereits oben genannt. Bevorzugte Antikörper gegen Fcγ-Rezeptoren sind Antikörper, die gegen die Ligandenbindungsstelle der niedrig-affinen Fcγ-Rezeptoren FcγRII und/oder FcγRIII gerichtet sind und deren Funktion blockieren.

Die eingesetzte Dosis des jeweiligen Antikörpers beträgt im allgemeinen ca. 0,1 bis ca. 4 mg/kg/Tag, vorzugsweise ca. 25 mg bis ca. 250 mg/Patient/Tag. Die übliche Behandlungsdauer des Patienten beträgt im allgemeinen ca. 1-9 Tage, vorzugsweise ca. 7-8 Tage.

Die Antikörper gemäß der vorliegenden Erfindung sind entweder polyklonal oder monoklonal. Die Herstellung der Antikörper erfolgt beispielsweise nach allgemein bekannten Methoden durch Immunisieren eines Säugetiers, beispielsweise eines Kaninchens, mit beispielsweise den entsprechenden Zellen, die das Antigen auf der Oberfläche exprimieren, wie z.B. T-Zellen, die den TCR/CD3-Komplex auf ihrer Oberfläche exprimieren (Howell, DG (1965), Vet. Rec 77:1391-1395; Hyde, RM (1967), Adv Appl Microbiol 9:39-67; Bailey, JM (1984) The production of antisera. In: Walker JM (ed) Methods on Molecular Biology, Vol. 1 Proteins, Seiten 295-300). Die hier aufgrund einer immunologischen Reaktion entstandenen polyklonalen Antikörper lassen sich anschließend nach allgemein bekannten Methoden leicht aus dem Blut isolieren und z. B. über Säulenchromatographie reinigen. Monoklonale Antikörper können beispielsweise nach der bekannten Methode von Köhler & Milstein (Köhler G, & Milstein C. (1975). Nature 256:495-497) hergestellt werden.

Die mitogene und/oder depletorische Aktivität der Antikörper läßt sich beispielsweise analog der Experimente gemäß Beispiel 1 und 2 der vorliegenden Erfindung feststellen. Beispiel 1 der vorliegenden Erfindung ist ein allgemeines Testsystem zur Beschreibung von in vivo mitogenen bzw. depletorischen Aktivitäten von Antikörpern. Die Messung der in vivo mitogenen bzw. depletorischen Aktivitäten der verwendeten Antikörper erfolgt über die Zahl der nachgewiesenen CD4⁺ T-Zellen (Vermehrung nach Gabe von mitogenen anti-TCR/CD3 Antikörpern und Verminderung nach Gabe von anti-TCR/CD3 Antikörpern mit depletorischer Aktivität).

Die folgenden Figuren und Beispiele sollen die Erfindung näher erläutern ohne sie zu beschränken:

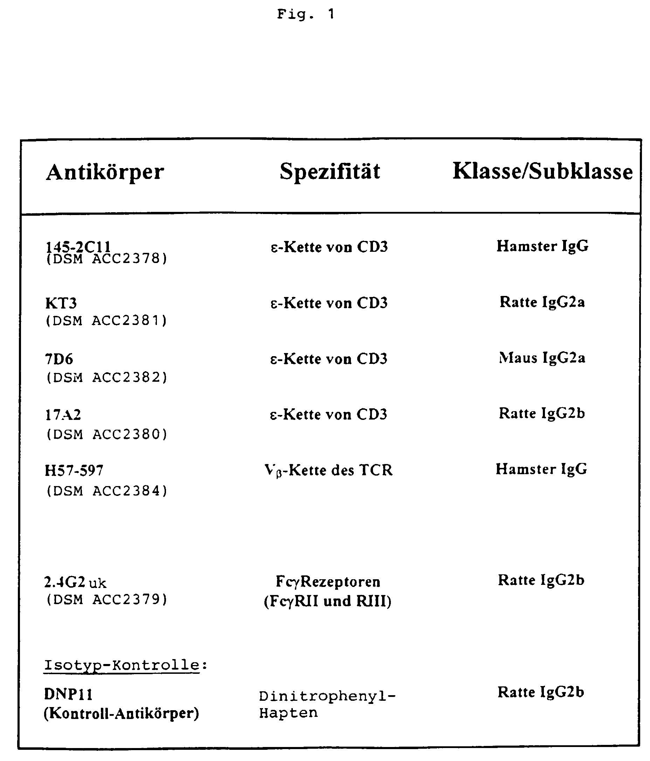

- **Figur 1**: ist eine Auflistung der verwendeten monoklonalen anti-TCR/CD3-Antikörper mit ihrer jeweiligen Spezifität und Bezeichnung der jeweiligen IgG-Subklasse.
- **Figur 2**: zeigt die mitogenen und depletorischen Aktivitäten von verschiedenen anti-TCR/CD3-Antikörpern, die sich in Spezifität und IgG-Subklasse unterscheiden.
- **Figur 3**: zeigt die Auswirkung der Blockade der niedrig-affinen Fcγ-Rezeptoren durch den Antikörper 2.4G2uk auf die mitogene und depletorische Aktivität der Antikörper 145-2C11 und KT3.
- **Figur 4A-C**: zeigt die Auswirkungen der Blockade der niedrig-affinen Fcγ-Rezeptoren durch den Antikörper 2.4G2uk auf die depletorische Aktivität der Antikörper 7D6, 17A2 und H57-597.

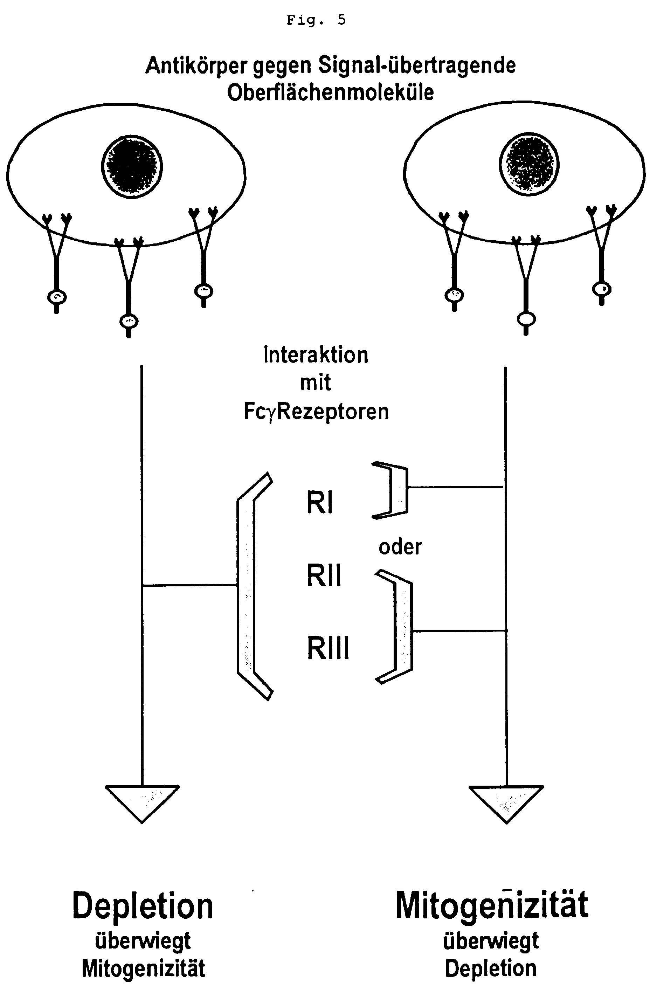

- **Figur 5**: zeigt schematisch, daß die depletorische oder mitogene Aktivität von Antikörpern gegen Signal-übertragende Oberflächenmoleküle durch deren Interaktionsmuster mit Fcγ-Rezeptoren festgelegt ist.

### Beispiele

### Beispiel 1

### Messung der mitogenen bzw. depletorischen Aktivitäten von verschiedenen Anti-TCR/CD3-Antikörper.

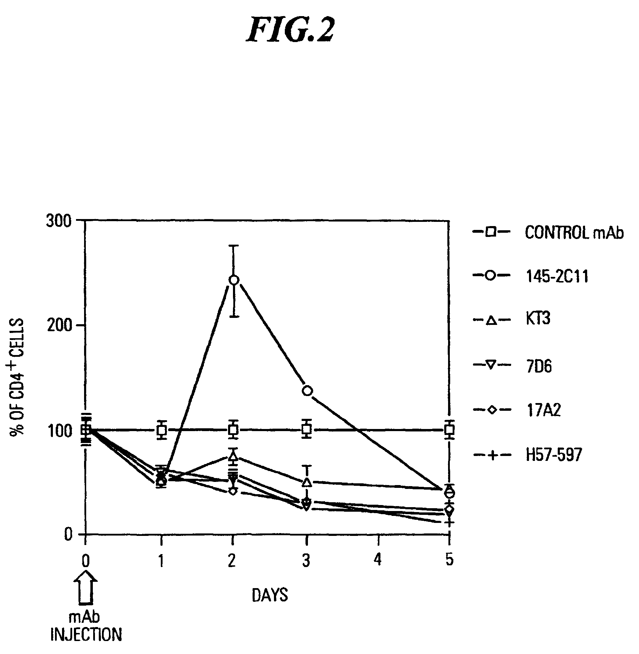

Sechs CBA/J-Mäuse pro Gruppe erhielten eine einmalige 400 µg Injektion der Antikörper 145-2C11, KT3, 7D6, 17A2, H57-597 oder DNP11 (Kontroll-Antikörper). An den in Figur 2 angegebenen Tagen wurden die Milzzellen der so behandelten Tiere gewonnen und mit einem käuflichen FITC (Fluorescein-Isothiocyanat) gekoppelten anti-CD4⁺-Antikörper im Fluoreszenz-aktivierten Zell-Analysator (FACS) untersucht.

Hierbei wurde festgestellt, daß die ausgeprägte depletorische Aktivität der Anti-körper 7D6, 17A2 und H57-597 während des Beobachtungszeitraums zu einer kontinuierlichen Elimination von CD4⁺-Zellen führt (Figur 2). Im Gegensatz dazu ist die depletorische Aktivität bei den Antikörpern 145-2C11 und KT3 weniger stark ausgeprägt und induziert statt dessen 48 Stunden nach Antikörpergabe die Proliferation von CD4⁺-Zellen (mitogene Aktivität).

### Beispiel 2

### Blockade der niedrig-affinen Fcγ-Rezeptoren

### 1. Wirkung auf mitogene Antikörper

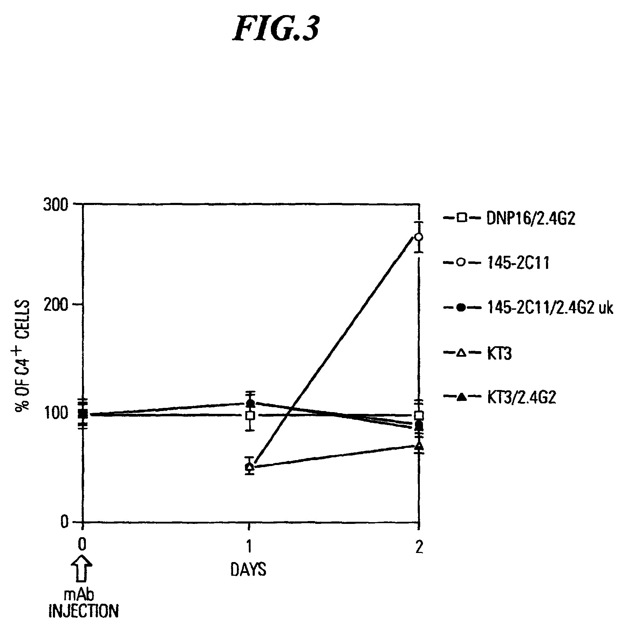

Sechs CBA/J-Mäuse pro Gruppe erhielten eine einmalige Injektion eine Antikörper-Cocktails bestehend aus 400 µg 145-2C11, KT3 oder DNP16 (Kontroll-Anti-körper) zusammen mit jeweils 1,0 mg des Antikörper 2.4G2uk. Die Milzzellen der so behandelten Tiere wurden an den in Figur 3 angegebenen Tagen gewonnen und mit einem käuflichen FITC-gekoppelten anti-CD4⁺-Antikörper im Fluoreszenz-aktivierten Zellanalysator (FACS) untersucht.

Die Versuchsergebnisse zeigen eindeutig, daß die mitogenen Antikörper 145-2C11 und KT3 ausschließlich mit den niedrig-affinen Fcγ-Rezeptoren interagieren und deren Blockade sowohl die depletorischen (24 Stunden nach Antikörpergabe) als auch die mitogenen Aktivitäten der beiden Antikörper 48 Stunden nach Anti-körpergabe vollständig aufhebt. Zur besseren Vergleichbarkeit sind die Ergebnisse der Depletionsexperimente ohne den Fcγ-Rezeptor-blockierenden Antikörper 2.4G2uk in Figur 3 mit aufgenommen.

### 2. Wirkung auf nicht-mitogene Antikörper

In einem weiteren Experiment erhielten sechs CBA/J-Mäuse pro Gruppe eine einmalige Injektion eines Antikörper-Cocktails bestehend aus 400 µg 7D6, 17A2, H57-597 oder DNP11 (Kontroll-Antikörper) zusammen mit jeweils 1,0 mg des Antikörpers 2.4G2uk. Milzzellen der so behandelten Tiere wurden an den angegeben Tagen nach Injektion des Antikörper-Cocktails gewonnen und mit einem FITC-gekoppelten anti-CD4⁺-Antikörper im Fluoreszenz-aktivierten Zell-Analysator (FACS) untersucht.

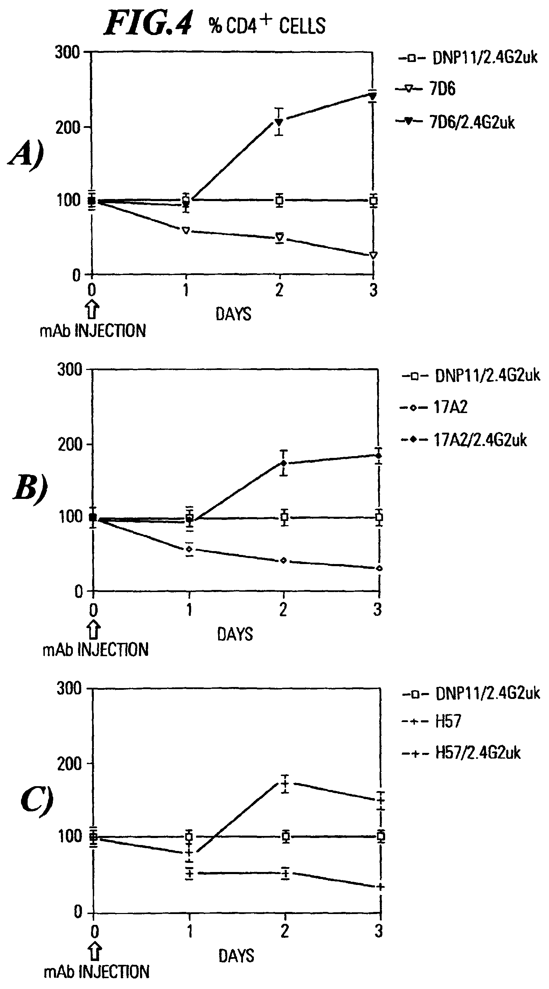

In diesem Experiment sind die nicht-mitogenen Antikörper 7D6, 17A2 und H57-597 nur noch in der Lage, den hoch-affinen Fcγ-Rezeptor RI zu rekrutieren. Das hat zur Folge, daß die Antikörper unter solchen Bedingungen überwiegend mitogene Aktivitäten vermitteln. Zur besseren Vergleichbarkeit sind die Ergebnisse der Depletionsexperimente ohne den Fcγ-Rezeptor-blockierenden Antikörper 2.4G2uk in Figur 4A-C mit aufgenommen.

## Patentansprüche

1. Verwendung eines oder mehrerer IgG-Antikörper in der Immuntherapie, dadurch gekennzeichnet, daß der oder die Antikörper gegen Antigene auf einer Zelloberfläche gerichtet sind, im wesentlichen mit den Fcγ-Rezeptoren I, II und III interagieren und eine im wesentlichen depletorische Aktivität zeigen.

2. Verwendung gemäß Anspruch 1, dadurch gekennzeichnet, daß der oder die Antikörper gegen Signal-übertragende Antigene auf einer Zelloberfläche, insbesondere gegen den TCR/CD3-Komplex gerichtet sind.

3. Verwendung nach Anspruch 1 oder 2, dadurch gekennzeichnet, daß der Antikörper ausgewählt ist aus dem Antikörper 7D6(DSM ACC2382), 17A2(DSM ACC2380) und/oder H57-597(DSM ACC2384).

4. Verwendung eines oder mehrerer IgG-Antikörper in der Immuntherapie, dadurch gekennzeichnet, daß der oder die Antikörper im wesentlichen mit den Fcγ-Rezeptoren II und III oder im wesentlichen mit dem Fcγ-Rezeptor I interagiert, gegen Signal-übertragende Oberflächenmoleküle, insbesondere gegen den TCR/CD3-Komplex, gerichtet sind und eine im wesentlichen mitogene Aktivität zeigen.

5. Verwendung nach Anspruch 4, dadurch gekennzeichnet, daß der Antikörper ausgewählt ist aus dem Antikörper 145-2C11(DSM ACC2378) und/oder KT3(DSM ACC2381).

6. Verwendung eines oder mehrerer Antikörper gegen mindestens einen Fcγ-Rezeptor in der Immuntherapie, dadurch gekennzeichnet, daß der oder die genannten Antikörper in Anwesenheit von im wesentlichen depletorisch wirkenden IgG-Antikörpern eine im wesentlichen mitogene Aktivität der depletorisch wirkenden Antikörper verursachen.

7. Verwendung nach Anspruch 6, dadurch gekennzeichnet, daß der oder die genannten Antikörper gegen die Ligandenbindungsstelle der Fcγ-Rezeptoren II und/oder III gerichtet sind.
